# EUROPEAN PATENT APPLICATION

(11) **EP 4 166 566 A1**
(43) Date of publication of application: **19.04.2023**
(21) Application number: 21820851.0
(22) Date of filing: 11.06.2021
(51) Int. Cl.: C07K 14/725, A61K 35/17, A61P 35/00

(54) **CHIMERIC ANTIGEN RECEPTOR AND USE THEREOF**

(30) Priority: 12.06.2020 CN 202010535848
(71) Applicant: Center for Excellence in Molecular Cell Science, Chinese Academy of Sciences, Shanghai 200031 (CN)
(72) Inventor: XU, Chenqi, Shanghai 200031 (CN); ZHOU, Qiuping, Shanghai 200031 (CN); WU, Wei, Shanghai 200031 (CN); HE, Xing, Shanghai 200031 (CN); SUN, He, Shanghai 200031 (CN)
(74) Representative: Canzler & Bergmeier Patentanwälte Partnerschaft mbB
(86) International application number: PCT/CN2021/099797
(87) International publication number: WO 2021/249549

(57) **Abstract**

Provided is a chimeric antigen receptor, comprising an extracellular domain, a transmembrane domain and an intracellular domain, which are connected in sequence, wherein the extracellular domain comprises an antigen recognition region and a hinge region; and one end of the intracellular domain which is connected to the transmembrane domain is connected to a CD3ε intracellular region. The chimeric antigen receptor can further improve the treatment effect of B-cell leukemia lymphoma in B-cell leukemia lymphoma treatment, and reduces inflammatory cytokines generated by means of macrophage monocyte activation by down-regulating cytokines of the chimeric antigen receptor, so that a cytokine storm can be prevented in an early stage, and the risk of neurotoxicity can be reduced. The treatment effect of solid tumors with high mesothelin expression is further improved in mesothelin-positive tumor treatment, and also, a cytokine storm can be prevented in an early stage and the risk of neurotoxicity can be reduced.

## Description

### FIELD OF DISCLOSURE

The present disclosure relates to the field of chimeric antigen receptor, and in particular, to a chimeric antigen receptor and use thereof.

### DESCRIPTION OF RELATED ARTS

The chimeric antigen receptor is referred to as CAR, and T cells which carries CAR targeting a specific tumor antigen are referred to as CAR-T cells. CAR-T therapy is widely used in tumor treatment, however, there are still many limitations. For example, the problems of cytokine storm, safety of neurotoxicity, and to-be-improved therapeutic effect when treating leukemia and solid tumor in the clinic, especially during the application of solid tumor immunotherapy.

At present, the methods for solving the cytokine storm (CRS) and neurotoxicity (NT) typically have limitations. Actemra (tocilizumab, IL-6R monoclonal antibody) of Roche was approved by FDA to manage CRS, where tocilizumab was approved for the first time by FDA for treating rheumatoid arthritis and can block the inflammatory response mediated by these cytokines. The disadvantage is that actemra elevates the IL-6 level in the blood of the patient, which can induce neurotoxicity through the blood-brain barrier; IL-1 inhibitor: in May 2018, two independent trials implemented by research teams of Italy's San Raffaele Hospital-San Raffaele Scientific Institute and the Memorial Sloan Kettering Cancer Center (MSKCC) demonstrated that CRS is induced by inflammatory molecule IL-1, therefore, the addition of IL-1 receptor antagonist Kineret (anakinra, drug for rheumatoid arthritis) in therapy can effectively control CRS by competitively inhibiting IL-1, which can also control neurotoxicity by virtue of its blood-brain barrier penetration ability. The limitation is that the tocilizumab and anakinra do not aim to prevent cells' CRS/NS in an early stage, but belong to emergency measures, therefore, the fatal risk exists in case of late remedy. In addition, it is not clear what is the optimal scheme for the side effects of early intervention currently.

An improvement of the CAR structure itself is believed to be a better scheme.

### SUMMARY OF THE PRESENT INVENTION

In view of disadvantages of the prior art, the present disclosure aims to provide a chimeric antigen receptor and use thereof.

In order to achieve the above objectives and other related purposes, a first aspect of the present disclosure provides a chimeric antigen receptor, including:
an extracellular domain, a transmembrane domain, and an intracellular domain which are connected in sequence,
where the extracellular domain includes an antigen recognition region and a hinge region;
and one end of the intracellular domain which is connected to the transmembrane domain is connected to a CD3ε intracellular region.

A second aspect of the present disclosure provides a polynucleotide sequence, which is selected from:
(1) a polynucleotide sequence encoding the aforementioned chimeric antigen receptor; and
(2) a complementary sequence of the polynucleotide sequence in (1).

A third aspect of the present disclosure provides a nucleic acid construct, including the aforementioned polynucleotide sequence.

In an embodiment, the nucleic acid construct is a vector.

In an embodiment, the nucleic acid construct is a lentivirus vector containing the aforementioned polynucleotide sequence, a replication initiation site, a 3'LTR, and a 5'LTR.

A fourth aspect of the present disclosure provides a lentivirus vector system, including the foregoing nucleic acid construct and a lentivirus vector auxiliary component.

A fifth aspect of the present disclosure provides a genetically modified T cell or a pharmaceutical composition including the genetically modified T cell, where the genetically modified T cell includes the above polynucleotide sequence or nucleic acid construct, or infects the aforementioned lentivirus vector system.

A sixth aspect of the present disclosure provides a use of the above chimeric antigen receptor, polynucleotide sequence, nucleic acid construct, or lentivirus vector system in any one or more of the following: (1) preparing T cell; (2) inhibiting T cell from secreting cytokines IFN-γ, IL-2, and TNF; (3) inhibiting T cell apoptosis; (5) enhancing T cell proliferation capacity; (6) improving T cell lethality; (7) promoting GrzB generation; and (8) promoting degranulation.

A seventh aspect of the present disclosure provides a use of the above chimeric antigen receptor, polynucleotide sequence, nucleic acid construct, lentivirus vector system, or genetically modified T cell in one or more of the following: (1) treating tumors; (2) inhibiting a generation of CRS during tumor treatment; and (4) inhibiting a generation of neurotoxicity during tumor treatment.

In an embodiment, the tumor is selected from leucocythemia or one or more of solid tumors.

As described above, the chimeric antigen receptor and the use thereof of the present disclosure have the following beneficial effects:

The chimeric antigen receptor of the present disclosure can further improve the treatment effect of B-cell leukemia lymphoma in B-cell leukemia lymphoma treatment, and reduce the activation of macrophage and monocyte through down-regulating cytokines resulting from the chimeric antigen receptor to decrease inflammatory cytokines, so that the cytokine storm can be prevented and the risk of neurotoxicity can be reduced in an early stage. In mesothelin-positive tumor therapy, the treatment effect of mesothelin high-expression solid tumors is further improved, and the prevention of cytokine storm and the reduced risk of neurotoxicity can be realized at an early stage.

### BRIEF DESCRIPTION OF THE DRAWINGS

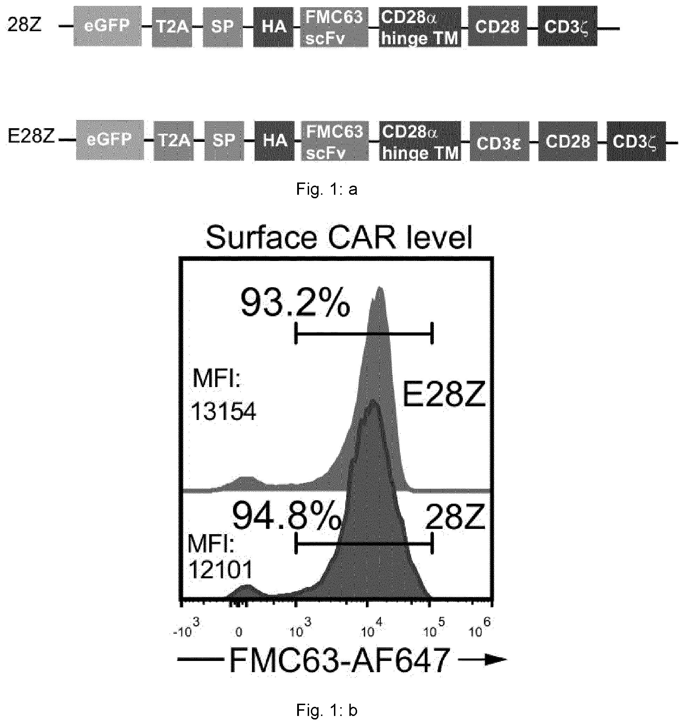

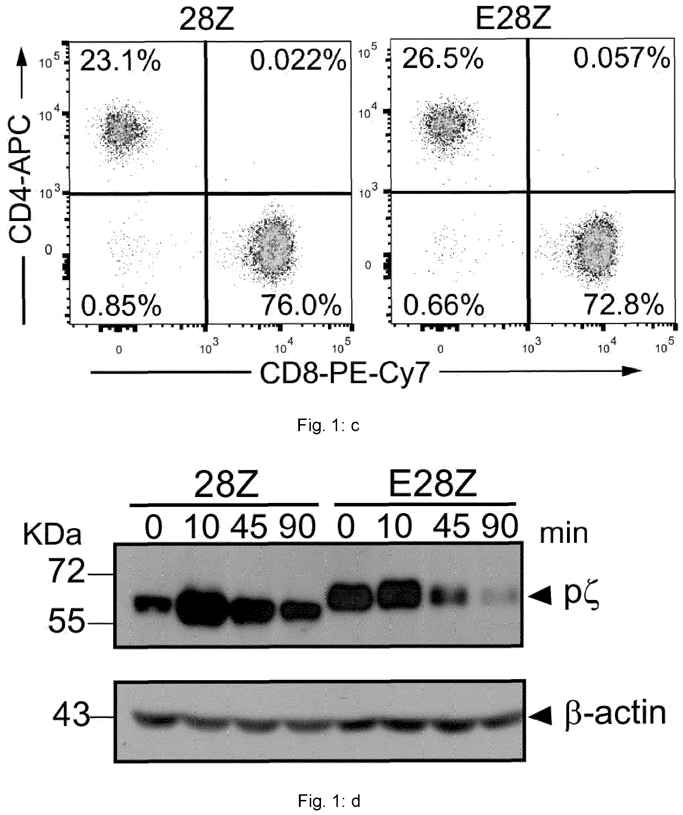

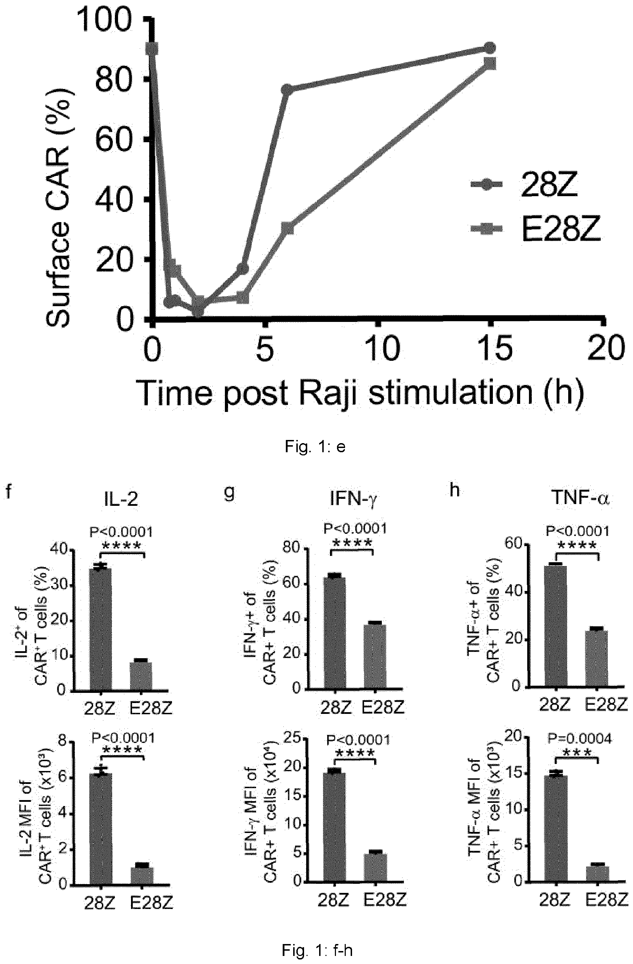

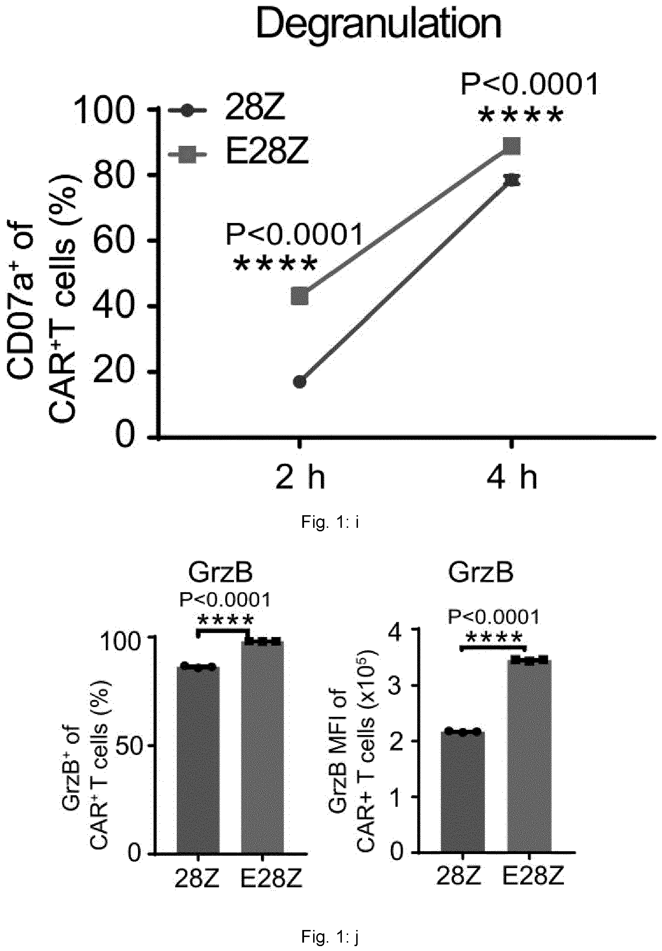

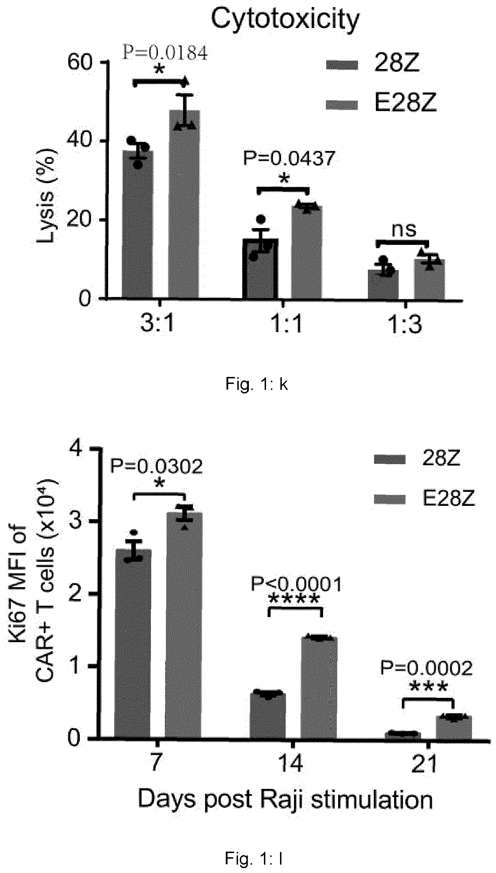

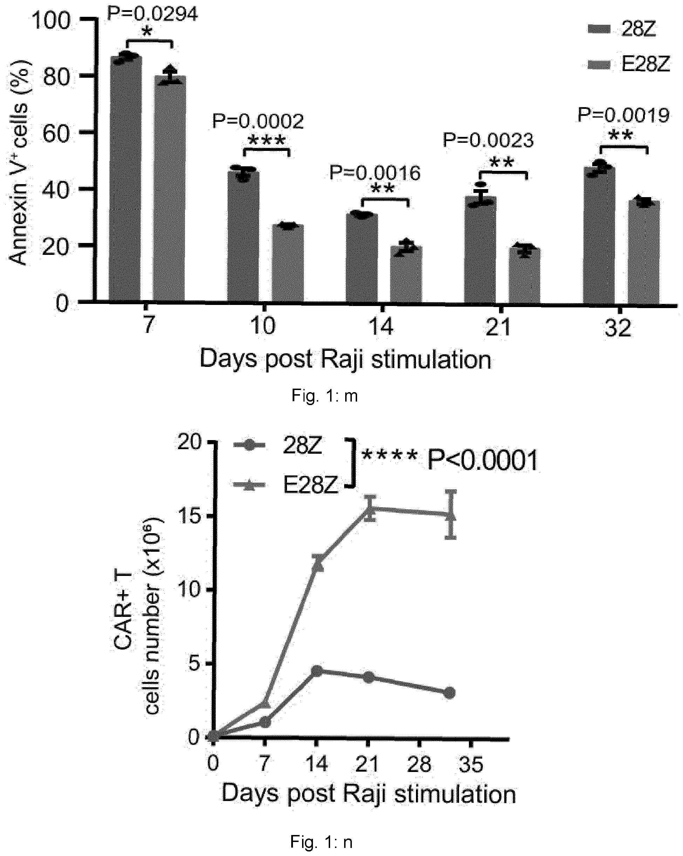

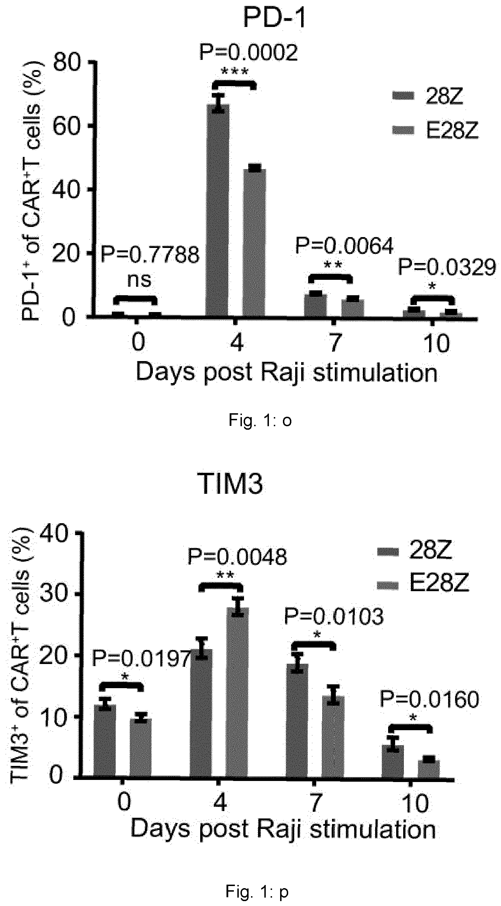

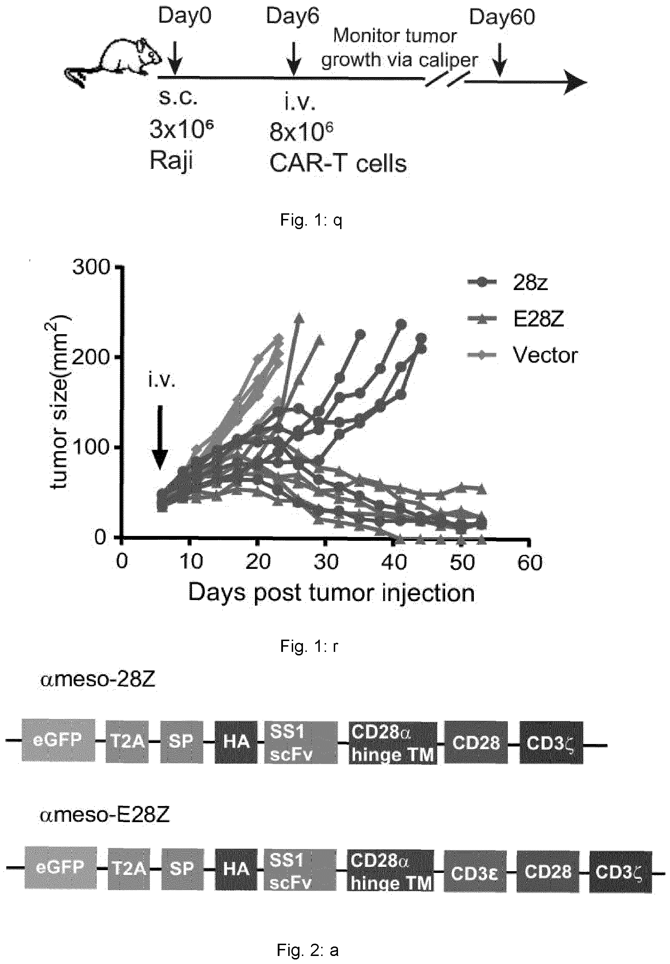

Fig. 1: shows a structural schematic diagram of 28Z CAR and E28Z CAR targeting CD19.
Fig. 1:b shows a flow diagram of anti-CD19 CAR on membrane surfaces of 28Z and E28Z CAR-T cells.
Fig. 1:c shows a flow diagram of CD4⁺ / CD8⁺ cell ratios of 28Z and E28Z CAR-T.
Fig. 1: d. shows CD3ζ phosphorylation levels of anti-CD19 28Z and E28Z CAR after CD19 antigenic stimulation.
Fig. 1: e. shows CAR levels on membrane surfaces of anti-CD19 28Z and E28Z CAR after CD19 antigenic stimulation.
Figs. 1: f-h. show cytokines levels of anti-CD19 28Z and E28Z CAR-T cells after CD19 antigenic stimulation.
Fig. 1: i. shows degranulation levels of anti-CD19 28Z and E28Z CAR-T cells after CD19 antigenic stimulation.
Fig. 1: j. shows granzyme efflux levels of anti-CD19 28Z and E28Z CAR-T cells after CD19 antigenic stimulation.
Fig. 1: k. shows cytotoxicity levels of anti-CD19 28Z and E28Z CAR-T cells against CD19⁺ tumor cell.
Fig. 1: I. shows proliferation capacities of anti-CD19 28Z and E28Z CAR-T cells after CD19 antigenic stimulation.
Fig. 1: m. shows apoptosis levels of anti-CD19 28Z and E28Z CAR-T cells after CD19 antigenic stimulation.
Fig. 1: n. shows survival levels of anti-CD19 28Z and E28Z CAR-T cells after CD19 antigenic stimulation.
Figs. 1: o-p. show exhaustion states of anti-CD19 28Z and E28Z CAR-T cells after CD19 antigenic stimulation.
Fig. 1: q. shows a flow chart of a Raji subcutaneous xenograft model where CAR-T cell is employed for treatment.
Fig. 1: r. shows a growth of Raji subcutaneous xenograft after CAR-T cell therapy.
Fig. 2: a. shows a structural schematic diagram of 28Z CAR and E28Z CAR targeting mesothelin.

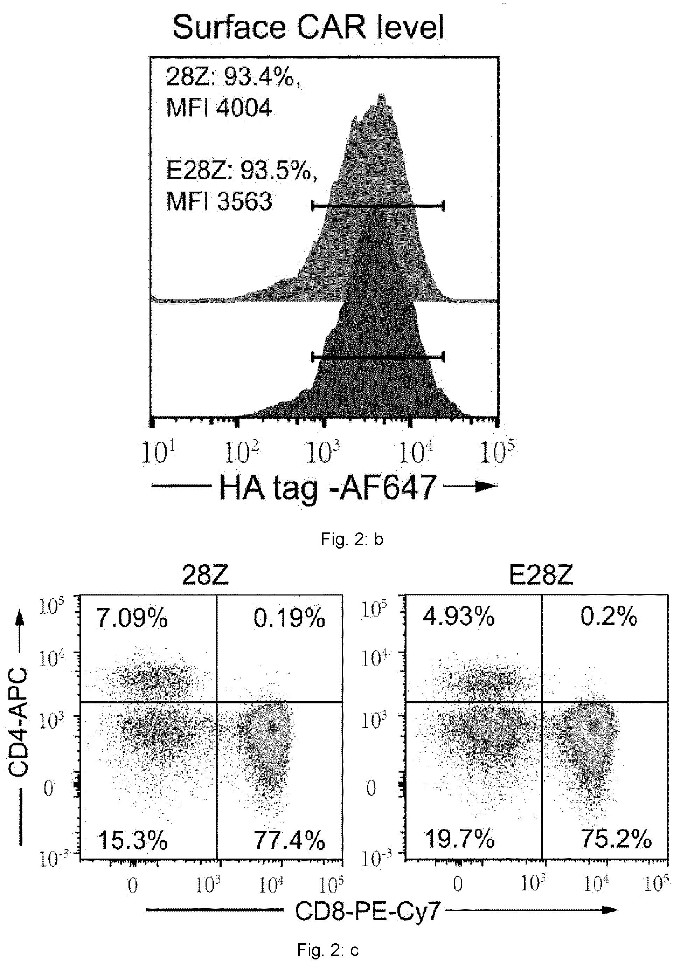

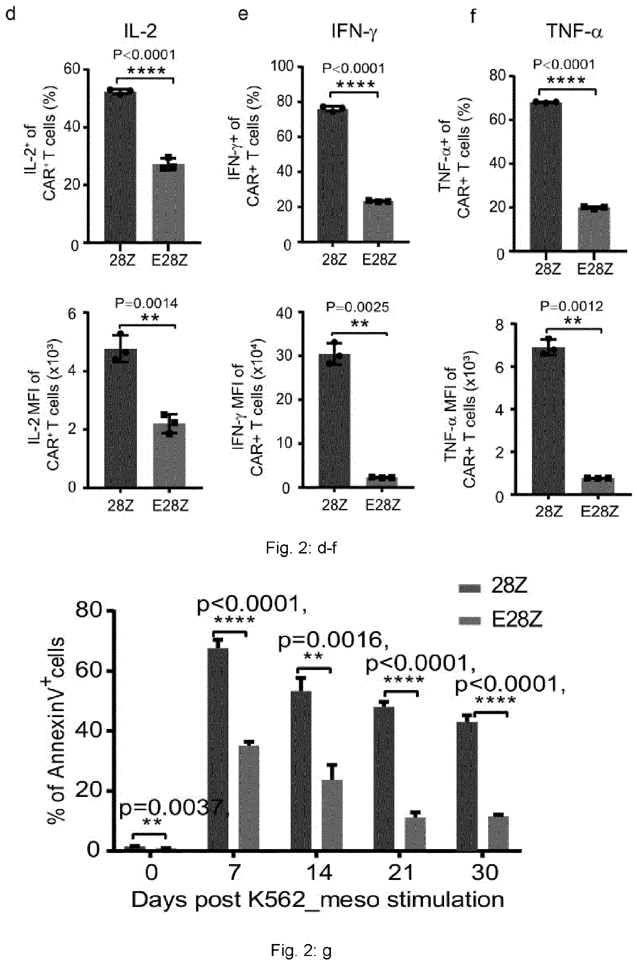

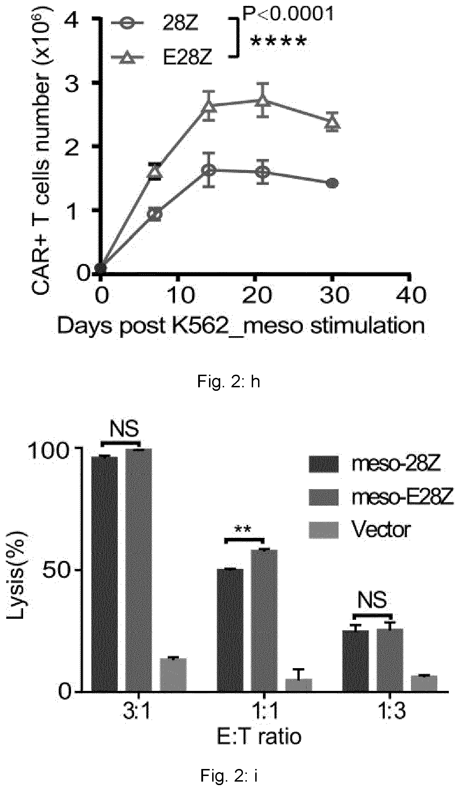

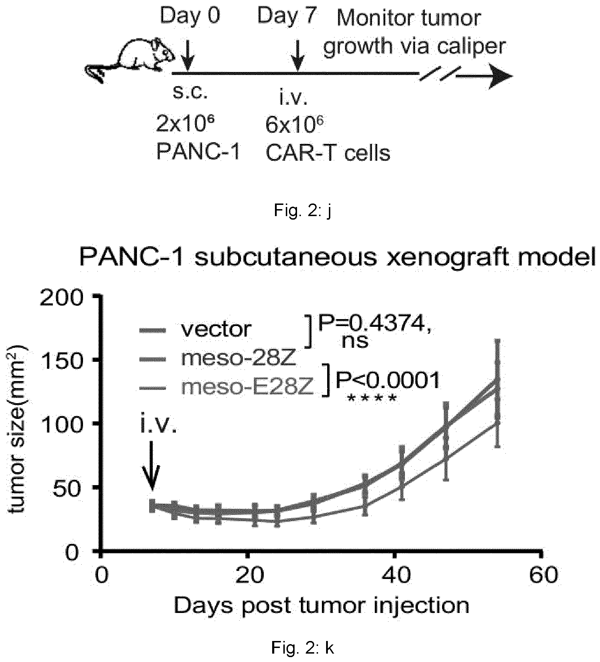

Fig. 2: b. shows a flow diagram of anti-mesothelin CAR on membrane surfaces of 28Z and E28Z CAR-T cells.
Fig. 2: c. shows a flow diagram of CD4⁺ / CD8⁺ cell ratios of anti-mesothelin 28Z and E28Z CAR-T.
Figs. 2: d-f. show cytokines levels of anti-mesothelin 28Z and E28Z CAR-T cells after mesothelin antigenic stimulation.
Fig. 2: g. shows apoptosis levels of anti-mesothelin 28Z and E28Z CAR-T cells after mesothelin antigenic stimulation.
Fig. 2: h. shows survival levels of anti-mesothelin 28Z and E28Z CAR-T cells after mesothelin antigenic stimulation.
Fig. 2: i. shows cytotoxicity levels of anti-mesothelin 28Z and E28Z CAR-T against mesothelin⁺ tumor cell.
Fig. 2: j. shows a flow chart of a subcutaneous xenograft model where anti-mesothelin CAR-T is employed for treating human PANC-1 pancreatic cancer cell line (highly expressing mesothelin).
Fig. 2: k. shows a growth of Raji subcutaneous xenograft after anti-mesothelin CAR-T cell therapy.

### DETAILED DESCRIPTION OF THE PREFERRED EMBODIMENTS

The chimeric antigen receptor of the present disclosure, includes:
an extracellular domain, a transmembrane domain, and an intracellular domain which are connected in sequence;
where the extracellular domain includes an antigen recognition region and a hinge region;
and one end of the intracellular domain which is connected to the transmembrane domain is connected to a CD3ε intracellular region.

Further, the amino acid sequence of CD3ε intracellular region is shown as SEQ ID NO:1; specifically:

KNRKAKAKPVTRGAGAGGRQRGQNKERPPPVPNPDYEPIRKGQRDLYSG
LNQRRI.

The intracellular domain includes the CD3ε intracellular region, a costimulatory signaling region, and a CD3ζ intracellular region which are connected in sequence.

In an embodiment, the costimulatory signaling region is selected from one or more of intracellular regions of CD27, CD28, CD134, 4-1BB, and ICOS.

In a further embodiment, the costimulatory signaling region is selected from CD28, whose killing capability is stronger.

The amino acid sequence of CD28 is shown as SEQ ID NO: 2; specifically: RSKRSRLLHSDYMNMTPRRPGPTRKHYQPYAPPRDFAAYRS.

In an embodiment, the amino acid sequence of CD3ζ intracellular region is shown as SEQ ID NO: 3, specifically:

RVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATK
DTYDALHMQALPPR.

In an embodiment, the antigen recognition region is a single-chain antibody against a tumor surface antigen, where the tumor surface antigen is selected from one or more of CD19, mesothelin, CD20, CD22, CD123, CD30, CD33, CD38, CD138, BCMA, Fibroblast activation protein (FAP), Glypican-3, CEA, EGFRvIII, PSMA, Her2, IL13Rα2, CD171, and GD2.

In an embodiment, the tumor surface antigen is selected from CD19 and mesothelin, which results in good targeting specificity.

The transmembrane domain is selected from CD28TM, CD4, CD8α, OX40, and H2-Kb.

In an embodiment, the transmembrane domain is selected from CD28TM. The amino acid sequence of CD28TM is shown as SEQ ID NO: 4, specifically: FWVLVWGGVLACYSLLVTVAFIIFWV.

In an embodiment, the single-chain antibody includes a light chain variable region and a heavy chain variable region.

In an embodiment, the light chain variable region and the heavy chain variable region are connected by a linker sequence.

In an embodiment, the single-chain antibody is selected from FMC63 and SS1.

FMC63 may be a commercially available product.

In an embodiment, the single-chain antibody includes a light chain variable region and a heavy chain variable region of a monoclonal antibody FMC63, and the light chain variable region and the heavy chain variable region are optionally connected by a linker.

In an embodiment, the single-chain antibody comprises a light chain variable region and a heavy chain variable region of a monoclonal antibody SS1, and the light chain variable region and the heavy chain variable region are optionally connected by a linker.

The nucleotide sequence of scFv of SS1 is shown as SEQ ID NO: 9, specifically:

CAGGTACAACTGCAGCAGTCTGGGCCTGAGCTGGAGAAGCCTG
GCGCTTCAGTGAAGATATCCTGCAAGGCTTCTGGTTACTCATTCACTGG
CTACACCATGAACTGGGTGAAGCAGAGCCATGGAAAGAGCCTTGAGTG
GATTGGACTTATTACTCCTTACAATGGTGCTTCTAGCTACAACCAGAAGT
TCAGGGGCAAGGCCACATTAACTGTAGACAAGTCATCCAGCACAGCCTA
CATGGACCTCCTCAGTCTGACATCTGAAGACTCTGCAGTCTATTTCTGT
GCAAGGGGGGGTTACGACGGGAGGGGTTTTGACTACTGGGGCCAAGG
GACCACGGTCACCGTCTCCTCAGGTGGAGGCGGTTCAGGCGGCGGTG
GCTCTAGCGGTGGTGGATCGGACATCGAGCTCACTCAGTCTCCAGCAA
TCATGTCTGCATCTCCAGGGGAGAAGGTCACCATGACCTGCAGTGCCA
GCTCAAGTGTAAGTTACATGCACTGGTACCAGCAGAAGTCAGGCACCTC
CCCCAAAAGATGGATTTATGACACATCCAAACTGGCTTCTGGAGTCCCA
GGTCGCTTCAGTGGCAGTGGGTCTGGAAACTCTTACTCTCTCACAATCA
GCAGCGTGGAGGCTGAAGATGATGCAACTTATTACTGCCAGCAGTGGA
GTAAGCACCCTCTCACGTACGGTGCTGGGACAAAGTTGGAAATCAAA

The amino acid sequence of scFv of SS1 is shown as SEQ ID NO: 10, specifically:

QVQLQQSGPELEKPGASVKISCKASGYSFTGYTMNWVKQSHGKSL

EWIGLITPYNGASSYNQKFRGKATLTVDKSSSTAYMDLLSLTSEDSAVYFCA

RGGYDGRGFDYWGQGTTVTVSSGGGGSGGGGSSGGGSDIELTQSPAIM
SASPGEKVTMTCSASSSVSYMHWYQQKSGTSPKRWIYDTSKLASGVPGR
FSGSGSGNSYSLTISSVEAEDDATYYCQQWSKHPLTYGAGTKLEIK

Further, the extracellular domain includes a signal peptide, which forms a connected signal peptide-antigen recognition region-hinge region.

In an embodiment, the amino acid sequence of the chimeric antigen receptor is shown as SEQ ID NO: 5 (Anti-CD19 E28Z CAR) or SEQ ID NO: 6 (Anti-mesothelin E28Z CAR), specifically;
SEQ ID NO: 5 (Anti-CD19 E28Z CAR):

MVSKGEELFTGVVPILVELDGDVNGHKFSVSGEGEGDATYGKLTLK
FICTTGKLPVPWPTLVTTLTYGVQCFSRYPDHMKQHDFFKSAMPEGYVQE
RTIFFKDDGNYKTRAEVKFEGDTLVNRIELKGIDFKEDGNILGHKLEYNYNS
HNVYIMADKQKNGIKVNFKIRHNIEDGSVQLADHYQQNTPIGDGPVLLPDNH
YLSTQSALSKDPNEKRDHMVLLEFVTAAGITLGMDELYKGSGEGRGSLLTC
GDVEENPGPMLLLVTSLLLCELPHPAFLLIPYPYDVPDYADIQMTQTTSSLS
ASLGDRVTISCRASQDISKYLNWYQQKPDGTVKLLIYHTSRLHSGVPSRFS
GSGSGTDYSLTISNLEQEDIATYFCQQGNTLPYTFGGGTKLEITGSTSGSGK
PGSGEGSTKGEVKLQESGPGLVAPSQSLSVTCTVSGVSLPDYGVSWIRQP
PRKGLEWLGVIWGSETTYYNSALKSRLTIIKDNSKSQVFLKMNSLQTDDTAI
YYCAKHYYYGGSYAMDYWGQGTSVTVSSAAAIEVMYPPPYLDNEKSNGTII
HVKGKHLCPSPLFPGPSKPFWVLVVVGGVLACYSLLVTVAFIIFWVKNRKA
KAKPVTRGAGAGGRQRGQNKERPPPVPNPDYEPIRKGQRDLYSGLNQRRI
RSKRSRLLHSDYMNMTPRRPGPTRKHYQPYAPPRDFAAYRSRVKFSRSA
DAPAYQQGQNQLYNELNLGRREEYDVLDKRRGRDPEMGGKPRRKNPQE
GLYNELQKDKMAEAYSEIGMKGERRRGKGHDGLYQGLSTATKDTYDALH
MQALPPR*

The nucleotide sequence corresponding to SEQ ID NO: 5 (Anti-CD19 E28Z CAR) is shown as SEQ ID NO: 7 (Anti-CD19 E28Z CAR), specifically:

ATGGTGAGCAAGGGCGAGGAGCTGTTCACCGGGGTGGTGCCCA
TCCTGGTCGAGCTGGACGGCGACGTAAACGGCCACAAGTTCAGCGTGT
CCGGCGAGGGCGAGGGCGATGCCACCTACGGCAAGCTGACCCTGAAG
TTCATCTGCACCACCGGCAAGCTGCCCGTGCCCTGGCCCACCCTCGTG
ACCACCCTGACCTACGGCGTGCAGTGCTTCAGCCGCTACCCCGACCAC
ATGAAGCAGCACGACTTCTTCAAGTCCGCCATGCCCGAAGGCTACGTC
CAGGAGCGCACCATCTTCTTCAAGGACGACGGCAACTACAAGACCCGC
GCCGAGGTGAAGTTCGAGGGCGACACCCTGGTGAACCGCATCGAGCT
GAAGGGCATCGACTTCAAGGAGGACGGCAACATCCTGGGGCACAAGCT
GGAGTACAACTACAACAGCCACAACGTCTATATCATGGCCGACAAGCAG
AAGAACGGCATCAAGGTGAACTTCAAGATCCGCCACAACATCGAGGAC
GGCAGCGTGCAGCTCGCCGACCACTACCAGCAGAACACCCCCATCGG
CGACGGCCCCGTGCTGCTGCCCGACAACCACTACCTGAGCACCCAGTC
CGCCCTGAGCAAAGACCCCAACGAGAAGCGCGATCACATGGTCCTGCT
GGAGTTCGTGACCGCCGCCGGGATCACTCTCGGCATGGACGAGCTGTA
CAAGGGAAGCGGAGAGGGCAGAGGAAGTCTGCTAACATGCGGTGACG
TCGAGGAGAATCCTGGACCTatgcttctcctggtgacaagccttctgctctgtgagttaccaca
cccagcattcctcctgatcccaTACCCCTACGACGTGCCCGACTACGCCgacatccag
atgacacagactacatcctccctgtctgcctctctgggagacagagtcaccatcagttgcagggcaagtca
ggacattagtaaatatttaaattggtatcagcagaaaccagatggaactgttaaactcctgatctaccatac
atcaagattacactcaggagtcccatcaaggttcagtggcagtgggtctggaacagattattctctcaccatt
agcaacctggagcaagaagatattgccacttacttttgccaacagggtaatacgcttccgtacacgttcgg
aggggggactaagttggaaataacaggctccacctctggatccggcaagcccggatctggcgagggat
ccaccaagggcgaggtgaaactgcaggagtcaggacctggcctggtggcgccctcacagagcctgtcc
gtcacatgcactgtctcaggggtctcattacccgactatggtgtaagctggattcgccagcctccacgaaag

ggtctggagtggctgggagtaatatggggtagtgaaaccacatactataattcagctctcaaatccagact
gaccatcatcaaggacaactccaagagccaagttttcttaaaaatgaacagtctgcaaactgatgacaca
gccatttactactgtgccaaacattattactacggtggtagctatgctatggactactggggtcaaggaacct
cagtcaccgtctcctcagcggccgcaattgaagttatgtatcctcctccttacctagacaatgagaagagca
atggaaccattatccatgtgaaagggaaacacctttgtccaagtcccctatttcccggaccttctaagcccttt
tgggtgctggtggtggttgggggagtcctggcttgctatagcttgctagtaacagtggcctttattattttctgggt
gaagaatagaaaggccaaggccaagcctgtgacacgaggagcgggtgctggcggcaggcaaaggg
gacaaaacaaggagaggccaccacctgttcccaacccagactatgagcccatccggaaaggccagc
gggacctgtattctggcctgaatcagagacgcatcaggagtaagaggagcaggctcctgcacagtgact
acatgaacatgactccccgccgccccgggcccacccgcaagcattaccagccctatgccccaccacgc
gacttcgcagcctatcgctccagagtgaagttcagcaggagcgcagacgcccccgcgtaccagcaggg
ccagaaccagctctataacgagctcaatctaggacgaagagaggagtacgatgttttggacaagagacg
tggccgggaccctgagatggggggaaagccgagaaggaagaaccctcaggaaggcctgtacaatga
actgcagaaagataagatggcggaggcctacagtgagattgggatgaaaggcgagcgccggagggg
caaggggcacgatggcctttaccagggtctcagtacagccaccaaggacacctacgacgcccttcacat
gcaggccctgccccctcgctaa

SEQ ID NO: 6 (Anti-mesothelin E28Z CAR):

MVSKGEELFTGVVPILVELDGDVNGHKFSVSGEGEGDATYGKLTLK
FICTTGKLPVPWPTLVTTLTYGVQCFSRYPDHMKQHDFFKSAMPEGYVQE
RTIFFKDDGNYKTRAEVKFEGDTLVNRIELKGIDFKEDGNILGHKLEYNYNS
HNVYIMADKQKNGIKVNFKIRHNIEDGSVQLADHYQQNTPIGDGPVLLPDNH
YLSTQSALSKDPNEKRDHMVLLEFVTAAGITLGMDELYKGSGEGRGSLLTC
GDVEENPGPMLLLVTSLLLCELPHPAFLLIPYPYDVPDYAQVQLQQSGPELE
KPGASVKISCKASGYSFTGYTMNWVKQSHGKSLEWIGLITPYNGASSYNQ
KFRGKATLTVDKSSSTAYMDLLSLTSEDSAVYFCARGGYDGRGFDYWGQ
GTTVTVSSGGGGSGGGGSSGGGSDIELTQSPAIMSASPGEKVTMTCSASS
SVSYMHWYQQKSGTSPKRWIYDTSKLASGVPGRFSGSGSGNSYSLTISSV

EAEDDATYYCQQWSKHPLTYGAGTKLEIKAAAIEVMYPPPYLDNEKSNGTII
HVKGKHLCPSPLFPGPSKPFWVLVVVGGVLACYSLLVTVAFIIFWVKNRKA
KAKPVTRGAGAGGRQRGQNKERPPPVPNPDYEPIRKGQRDLYSGLNQRRI
RSKRSRLLHSDYMNMTPRRPGPTRKHYQPYAPPRDFAAYRSRVKFSRSA
DAPAYQQGQNQLYNELNLGRREEYDVLDKRRGRDPEMGGKPRRKNPQE
GLYNELQKDKMAEAYSEIGMKGERRRGKGHDGLYQGLSTATKDTYDALH
MQALPPR*

The nucleotide sequence corresponding to SEQ ID NO: 6 (Anti-mesothelin E28Z CAR) is shown as SEQ ID NO: 8 (Anti-mesothelin E28Z CAR), specifically:

ATGGTGAGCAAGGGCGAGGAGCTGTTCACCGGGGTGGTGCCCA
TCCTGGTCGAGCTGGACGGCGACGTAAACGGCCACAAGTTCAGCGTGT
CCGGCGAGGGCGAGGGCGATGCCACCTACGGCAAGCTGACCCTGAAG
TTCATCTGCACCACCGGCAAGCTGCCCGTGCCCTGGCCCACCCTCGTG
ACCACCCTGACCTACGGCGTGCAGTGCTTCAGCCGCTACCCCGACCAC
ATGAAGCAGCACGACTTCTTCAAGTCCGCCATGCCCGAAGGCTACGTC
CAGGAGCGCACCATCTTCTTCAAGGACGACGGCAACTACAAGACCCGC
GCCGAGGTGAAGTTCGAGGGCGACACCCTGGTGAACCGCATCGAGCT
GAAGGGCATCGACTTCAAGGAGGACGGCAACATCCTGGGGCACAAGCT
GGAGTACAACTACAACAGCCACAACGTCTATATCATGGCCGACAAGCAG
AAGAACGGCATCAAGGTGAACTTCAAGATCCGCCACAACATCGAGGAC
GGCAGCGTGCAGCTCGCCGACCACTACCAGCAGAACACCCCCATCGG
CGACGGCCCCGTGCTGCTGCCCGACAACCACTACCTGAGCACCCAGTC
CGCCCTGAGCAAAGACCCCAACGAGAAGCGCGATCACATGGTCCTGCT
GGAGTTCGTGACCGCCGCCGGGATCACTCTCGGCATGGACGAGCTGTA
CAAGGGAAGCGGAGAGGGCAGAGGAAGTCTGCTAACATGCGGTGACG
TCGAGGAGAATCCTGGACCTatgcttctcctggtgacaagccttctgctctgtgagttaccaca

cccagcattcctcctgatcccaTACCCCTACGACGTGCCCGACTACGCCCAGGTAC
AACTGCAGCAGTCTGGGCCTGAGCTGGAGAAGCCTGGCGCTTCAGTGA
AGATATCCTGCAAGGCTTCTGGTTACTCATTCACTGGCTACACCATGAA
CTGGGTGAAGCAGAGCCATGGAAAGAGCCTTGAGTGGATTGGACTTAT
TACTCCTTACAATGGTGCTTCTAGCTACAACCAGAAGTTCAGGGGCAAG
GCCACATTAACTGTAGACAAGTCATCCAGCACAGCCTACATGGACCTCC
TCAGTCTGACATCTGAAGACTCTGCAGTCTATTTCTGTGCAAGGGGGGG
TTACGACGGGAGGGGTTTTGACTACTGGGGCCAAGGGACCACGGTCAC
CGTCTCCTCAGGTGGAGGCGGTTCAGGCGGCGGTGGCTCTAGCGGTG
GTGGATCGGACATCGAGCTCACTCAGTCTCCAGCAATCATGTCTGCATC
TCCAGGGGAGAAGGTCACCATGACCTGCAGTGCCAGCTCAAGTGTAAG
TTACATGCACTGGTACCAGCAGAAGTCAGGCACCTCCCCCAAAAGATG
GATTTATGACACATCCAAACTGGCTTCTGGAGTCCCAGGTCGCTTCAGT
GGCAGTGGGTCTGGAAACTCTTACTCTCTCACAATCAGCAGCGTGGAG
GCTGAAGATGATGCAACTTATTACTGCCAGCAGTGGAGTAAGCACCCTC
TCACGTACGGTGCTGGGACAAAGTTGGAAATCAAAgcggccgcaattgaagttat
gtatcctcctccttacctagacaatgagaagagcaatggaaccattatccatgtgaaagggaaacaccttt
gtccaagtcccctatttcccggaccttctaagcccttttgggtgctggtggtggttgggggagtcctggcttgct
atagcttgctagtaacagtggcctttattattttctgggtgaagaatagaaaggccaaggccaagcctgtga
cacgaggagcgggtgctggcggcaggcaaaggggacaaaacaaggagaggccaccacctgttccc
aacccagactatgagcccatccggaaaggccagcgggacctgtattctggcctgaatcagagacgcatc
aggagtaagaggagcaggctcctgcacagtgactacatgaacatgactccccgccgccccgggcccac
ccgcaagcattaccagccctatgccccaccacgcgacttcgcagcctatcgctccagagtgaagttcagc
aggagcgcagacgcccccgcgtaccagcagggccagaaccagctctataacgagctcaatctaggac
gaagagaggagtacgatgttttggacaagagacgtggccgggaccctgagatggggggaaagccgag
aaggaagaaccctcaggaaggcctgtacaatgaactgcagaaagataagatggcggaggcctacagt
gagattgggatgaaaggcgagcgccggaggggcaaggggcacgatggcctttaccagggtctcagtac
agccaccaaggacacctacgacgcccttcacatgcaggccctgccccctcgctaa

The above-mentioned parts of the chimeric antigen receptor in the present disclosure may be connected directly with each other or connected through the linker sequence. The linker sequence may be any linker sequence suitable for antibody and known in this field, for example, the linker sequence may contain G and S. Generally speaking, the linker may contain one or more repeated motifs. For example, the motif may be GGGS, GGGGS, SSSSG, GSGSA, and GGSGG. In an embodiment, the motifs of the linker sequence are adjacent to each other and no amino acid residue is inserted in the repeated motifs. The linker sequence may include 1, 2, 3, 4, or 5 repeated motifs, and the length of the linker may be 3-25 amino acid residues, such as 3-15, 5-15, or 10-20 amino acid residues. In some embodiments, the linker sequence may contain multiple glycines. The number of glycine in the linker sequence is not particularly limited, and may be 2-20, for example, 2-15, 2-10, or 2-8. Except for glycine and serine, the linker may also contain other known amino acid residues, such as alanine (A), leucine (L), threonine (T), glutamic acid (E), phenylalanine (F), arginine (R), and glutamine (Q).

It should be understood that a restriction enzyme site is usually needed in gene cloning, therefore, one or more unrelated residues will be introduced to a terminal of the amino acid sequence to be expressed, however, this operation has no effect on the activity of the targeted sequence. In order to construct a fusion protein, promote the expression of a recombinant protein, obtain the recombinant protein that is secreted out of the host automatically, or purify the recombinant protein, it is usually required to add some amino acids to an N-terminal, a C-terminal, or other proper regions in the recombinant protein, such as, including but not limited to, proper adaptor peptide, signal peptide, leader peptide, etc. Therefore, the amino terminal or carboxyl terminal of the fusion protein (i.e. the CAR) of the present disclosure may include one or more polypeptide fragments to serve as protein tags. Any suitable tag may be used herein. For example, the tag may be FLAG, HA, HA1, c-Myc, Poly-His, Poly-Arg, Strep-Tagll, AU1, EE, T7, 4A6, ε, B, gE, and Ty1. These tags may be used for protein purification.

The polynucleotide sequence provided by the present disclosure is selected from:
(1) a polynucleotide sequence encoding the aforementioned chimeric antigen receptor; and
(2) a complementary sequence of the polynucleotide sequence in (1).

The polynucleotide sequence of the present disclosure may be in the form of DNA or RNA. DNA includes cDNA, genomic DNA, or synthetic DNA. The DNA may be single-stranded or double-stranded. The DNA may be a coding strand or a non-coding strand. The present disclosure also includes a variant of the polynucleotide sequence which encodes the fusion protein, i.e., a nucleotide sequence encoding the same amino acid sequence but having a different nucleotide sequence.

The polynucleotide sequences described herein may be obtained by PCR amplification. Specifically, primers can be designed according to the nucleotide sequences disclosed herein, in particular, according to open reading frame sequences, and related sequences can be obtained by using a commercially available cDNA library or a cDNA library prepared according to conventional methods known to a person skilled in the art as a template. When the sequence is relatively long, two or more PCR amplifications are needed, and then the amplified fragments are spliced together in the correct order.

The nucleic acid construct provided by the present disclosure includes the above polynucleotide sequence.

The nucleic acid construct further includes one or more regulatory sequences operatively connected to the aforementioned polynucleotide sequence. The coding sequence of the CAR of the present disclosure can be operated in a variety of ways to ensure the expression of the protein. The nucleic acid construct may be operated according to different expression vectors or requirements of the expression vector before inserting the nucleic acid construct into the vector. Altering polynucleotide sequences by using the recombinant DNA method is known in the art.

The regulatory sequence may be a suitable promoter sequence. The promoter sequence is usually operatively connected to the coding sequence of the protein to be expressed. The promoter may be any nucleotide sequence that shows transcriptional activity in the selected host cell, including mutated, truncated, and hybrid promoters, and may be obtained from genes encoding extracellular or intracellular polypeptides which are homologous or heterologous to the host cell.

The regulatory sequence may also be a suitable transcription terminator sequence, i.e., the sequence identified by the host cell to terminate the transcription. The terminator sequence is operatively connected to the 3' terminal of the nucleotide sequence encoding the polypeptide. Any terminator with functionality in the selected host cell may be used in the present disclosure.

The regulatory sequence may also be a suitable leader sequence, i.e., an untranslated region for mRNA which is important to the translation of a host cell. The leader sequence is operably linked to the 5' terminal of the nucleotide sequence encoding the polypeptide. Any leader sequence with functionality in the selected host cell may be used in the present disclosure.

In an embodiment, the nucleic acid construct is a vector.

The expression of the polynucleotide sequence encoding the CAR is typically achieved by operably linking the polynucleotide sequence encoding the CAR to the promoter and incorporating the construct into the expression vector. The vector may be suitable for the replication and integration of eukaryotic cells. Typical cloning vectors include transcription and translation terminators, initiating sequences, and promoters that can be used for modulating the expression of desired nucleic acid sequences.

The polynucleotide sequence encoding the CAR of the present disclosure may be cloned into many types of vectors, for example, plasmid, phagemid, phage derivative, animal virus, and cosmid. Further, the vector is an expression vector. The expression vector may be provided to the cell in the form of a virus vector. Virus vector technology is known in the art. Viruses that can be used as vectors include but are not limited to, retroviruses, adenoviruses, adeno-associated viruses, herpesviruses, and lentivirus. Generally, a suitable vector includes a replication origin, a promoter sequence, a convenient restriction enzyme site, and one or more selectable tags that function in at least one organism.

In an embodiment, the nucleic acid construct is a lentivirus vector containing the replication origin, 3'LTR, 5'LTR, and the foregoing polynucleotide sequence.

One example of a suitable promoter is an immediate early cytomegalovirus (CMV) promoter sequence. The promoter sequence is a strong constitutive promoter sequence capable of driving high-level expression of any polynucleotide sequence operably linked thereto. Another example of a suitable promoter is an elongation factor-1α (EF-1α). However, other constitutive promoter sequences may also be used, including but not limited to, simian virus 40 (SV40) early promoter, mouse mammary tumor virus (MMTV), human immunodeficiency virus (HIV) long terminal repeated (LTR) promoter, MoMuLV promoter, avian leukosis virus promoter, EB virus immediate early promoter, Rous sarcoma virus promoter, and human gene promoter (including but not limited to actin promoter, myosin promoter, heme promoter, and creatine kinase promoter). Further, the use of an inducible promoter may also be considered. The use of an inducible promoter provides a molecular switch that is capable of starting the expression of the polynucleotide sequence operably linked to the inducible promoter upon expression, and terminating the expression when the expression is undesirable. Examples of inducible promoters include, but are not limited to, metallothionein promoters, glucocorticoid promoters, progesterone promoters, and tetracycline promoters.

In order to assess the expression of CAR polypeptides or portions thereof, the expression vectors introduced into the cells may also include one or both of the selectable marker genes or reporter genes to facilitate the identification and selection of expression cells from a cell population sought to be transfected or infected by a viral vector. In other aspects, the selectable markers may be carried on a single DNA segment and used for co-transfection procedures. The flanking regions of both selectable markers and reporter genes may have appropriate regulatory sequences to enable them to be expressed in host cells. Useful selectable markers include, for example, antibiotic resistance genes (such as nco).

Reporter genes are used for identifying potential transfected cells and evaluating the functionality of regulatory sequences. After DNA has been introduced into recipient cells, the expression of the reporter gene is then measured at an appropriate time. Appropriate reporter genes may contain genes encoding luciferase, β-galactosidase, chloramphenicol acetyltransferase (CAT), secreted alkaline phosphatase (SAP), or green fluorescent protein gene (GFP). Appropriate expression systems are well-known and can be prepared using known techniques or commercially available.

Methods of introducing genes into cells and methods for gene overexpression in cells are known in the art. The vector may be easily introduced into a host cell, e.g., a mammal, a bacterium, a yeast, or an insect cell, by any method in the art. For example, the expression vector may be transferred into a host cell by physical, chemical, or biological methods.

Physical methods of introducing polynucleotides into host cells include calcium phosphate precipitation, lipid transfection, particle bombardment, micro-injection, electroporation, etc. Biological methods of introducing polynucleotides of interest into host cells include the use of DNA and RNA vectors. Chemical methods of introducing polynucleotides into host cells include colloidal dispersion systems, such as macromolecular complexes, nanocapsules, microspheres, and beads; and lipid-based systems, including oil-in-water emulsions, micelles, mixed micelles, and liposomes.

Biological methods of introducing polynucleotides into host cells further include the use of viral vectors, in particular lentivirus vectors, which have become the most widely used methods of inserting genes into mammals, such as human cells. Other viral vectors may be derived from lentivirus, poxvirus, herpes simplex virus I, adenovirus, adeno-associated virus, etc. Many virus-based systems have been developed for transferring genes into mammal cells. For example, lentivirus provides a convenient platform for gene delivery systems. The selected genes can be inserted into the vector and packaged into lentivirus particles using techniques known in the art, and the obtained recombinant virus may then be separated and transferred to a subject cell in vivo or in vitro. Many retrovirus systems are known in the art. In some embodiments, an adenovirus vector is used. Many adenovirus vectors are known in the art. In an embodiment, a lentivirus vector is used.

The lentivirus vector system provided by the present disclosure includes the foregoing nucleic acid construct and a lentivirus vector auxiliary component.

The lentivirus auxiliary component includes a lentivirus packaging plasmid and a cell line.

The lentivirus vector system is formed by packaging the nucleic acid construct through a virus with the aid of the lentivirus packaging plasmid and the cell line. The method for constructing the lentivirus vector system is a common method in the art.

The present disclosure provides a method for activation of T cells in vitro, including: infecting the T cells with the aforementioned lentivirus.

The CAR-T cells of the present disclosure can undergo robust in vivo T cell expansion, persist in blood and bone marrow, and form specific memory T cells. The CAR-T cells of the present disclosure can differentiate into a central memory-like state in vivo after encountering and then eliminating target cells expressing alternative antigens.

The present disclosure also provides a cell therapy, where T cells are genetically modified to express CAR described herein and CAR-T cells are injected into subjects in need thereof. The injected cells can kill tumor cells in subjects. Unlike antibody therapy, CAR-T cell can replicate in vivo, thus producing long-term control of the tumor.

The antitumor immune response caused by CAR-T cells may be active or passive immune response. In addition, the CAR-mediated immune response can be part of adoptive immunotherapy, where the CAR-T cell induces an immune response specifically corresponding to the antigen-binding moiety in CAR.

Cancer that can be treated may be a non-solid tumor, such as a blood tumor (such as leukemia and lymphoma). In particular, the diseases that can adopt the CAR and its coding sequence, nucleic acid construct, expression vector, virus, and CAR-T cell of the present disclosure for treatment are CD19-mediated diseases, especially CD19-mediated hematologic tumors.

Specifically, "CD19-mediated diseases" include, but are not limited to, leukemia and lymphoma, such as B-cell lymphoma, mantle cell lymphoma, acute lymphoblastic leukemia (ALL), chronic lymphocytic leukemia (CLL), hairy cell leukemia (HCL), and acute myeloid leukemia (AML).

The present disclosure provides the genetically modified T cell or pharmaceutical composition including the genetically modified T cell, where the genetically modified T cell includes the above polynucleotide sequence or nucleic acid construct, or infects the aforementioned lentivirus vector system.

The CAR-modified T cells of the present disclosure may be separately administrated or administrated as the pharmaceutical composition in combination with a diluent and/or other components (such as related cytokines or cell population). Briefly stated, the pharmaceutical composition of the present disclosure may include CAR-T cells described herein, which can be in combination with one or more medically or physiologically acceptable vectors, diluents, or excipients. Such composition may include buffer solutions, such as neutral buffer brine and sulfate buffer brine; carbohydrates, such as glucose, mannose, sucrose or glucan, and mannitol; protein; polypeptides or amino acids, such as glycine; antioxidant; chelating agent, such as EDTA or glutathione; adjuvant (such as aluminium hydroxide); and antiseptic agent.

The pharmaceutical composition of the present disclosure may be administrated in the way required by diseases to be treated (or prevented). The amount and frequency of administration depend on such factors, for example, the patient's symptoms, and disease type and severity.

When the "immunologically effective dosage", "antitumor effective dosage", "tumor-inhibitory effective dosage" or "therapeutic dosage" are indicated, the precise dosage of the composition of the present disclosure to be administrated depends on physicians after taking into account the age, weight, tumor size, infection or metastasis degree of the patient (subject) and individual differences. It may be indicated that the pharmaceutical composition containing the T cells described herein may be administrated at the dose of 10⁴ to 10⁹ cells/kg body weight, preferably 10⁵ to 10⁶ cells/kg body weight. The composition containing the T-cell may also be administrated multiple times at these doses. The cells may be administered by injection techniques known in the field of immunotherapy. The optimal dose and therapy scheme for a particular patient may be readily determined by a person skilled in the medical field by monitoring the signs of disease of the patient which facilitates the adjustment of the treatment.

The administration of the composition may be performed in any convenient manner, including spray, injection, swallowing, infusion, implantation, or transplantation. The compositions described herein may be administrated to patients subcutaneously, intracutaneously, intratumorally, nodally, intraspinally, intramuscularly, intravenously, or intraperitoneally. In an embodiment, the composition containing the T cell of the present disclosure is administrated to patients through intracutaneous or subcutaneous injection. In another embodiment, the composition containing the T cell of the present disclosure is administrated by intravenous injection. The composition containing the T cell may be injected directly into a tumor, lymph gland, or infection site.

In some embodiments of the present disclosure, the CAR-T cells or the composition thereof of the present disclosure may be combined with other therapies known in the art. The therapies include, but are not limited to, chemotherapy, radiotherapy, and immunosuppressants. For example, the CAR-T cells or the composition thereof of the present disclosure may be administrated in combination with various radiotherapy formulations, including cyclosporine, thiopurine, methotrexate, mycophenolate mofetil, FK506, fludarabine, rapamycin, mycophenolic acid, etc. In a further embodiment, the composition containing the T cell of the present disclosure is administered to the patient (e.g., before, during, or after) with bone marrow transplantation; a chemotherapeutic agent, such as fludarabine; external radiotherapy (XRT), cyclophosphamide or antibody, such as OKT3; or CAMPATH's T cell ablation therapy.

"Anti-tumor effect" herein refers to a biological effect that may be represented as a reduction in tumor size, a reduction in the number of tumor cells, a reduction in the number of metastasis, an increase in expected lifetime, or an alleviation of various physiological symptoms associated with cancer.

"Patient", "object", "individual", and the like may be used interchangeably herein to refer to a living organism, such as a mammal, that may cause an immune response. Examples include, but are not limited to, humans, dogs, cats, mice, rats, and transgenic species thereof.

The use of the aforementioned chimeric antigen receptor, polynucleotide sequence, nucleic acid construct, and lentivirus vector system in any one or more of the following: (1) preparing T cells; (2) inhibiting T cells from secreting cytokines IFN-γ, IL-2, and TNF; (3) inhibiting T cell apoptosis; (5) enhancing T cell proliferation capacity; (6) improving T cell lethality; (7) promoting GRZB generation; and (8) promoting degranulation.

The use of the above chimeric antigen receptor, polynucleotide sequence, nucleic acid structure, lentivirus vector system, or genetically modified T cell in one or more of the following: (1) treating tumors; (3) inhibiting a generation of CRS during tumor treatment; and (4) inhibiting a generation of neurotoxicity during tumor treatment.

In an embodiment, the tumor is selected from leucocythemia or one or more of solid tumors.

In an embodiment, the tumor is selected from B-cell lymphoma, mantle cell lymphoma, acute lymphoblastic leukemia (ALL), chronic lymphocytic leukemia (CLL), hairy cell leukemia (HCL), and acute myeloid leukemia (AML).

The implementations of the present disclosure are described below through specific embodiments. Other advantages and effects of the present disclosure will be readily apparent to those skilled in the art from the content disclosed in the description. The present disclosure can also be implemented or applied through other different specific embodiments, and various details in the description can also be modified or changed without departing from the spirit of the present disclosure based on different viewpoints and applications.

Before further describing the specific embodiments of the present disclosure, it should be understood that the protection scope of the present disclosure is not limited to the specific embodiments described below. It should also be understood that the terms used in the embodiments of the present disclosure are intended to describe specific embodiments, rather than limiting the protection scope of the present disclosure. As used herein, "a", "an", and "the" in the singular form include plural forms, unless otherwise stated herein.

When a numerical range is provided in an embodiment, it should be understood that any value between two endpoints of each numerical range and the two endpoints may be selected unless otherwise indicated by the present disclosure. Unless otherwise defined, all technical and scientific terms in the present disclosure have the same meaning as commonly understood by those skilled in the art. In addition to the specific methods, apparatuses, and materials used in the embodiments, any method, apparatus, and material of the prior art similar to or equivalent to those described in the embodiments of the present disclosure may also be used.

Unless otherwise specified, the experimental methods, detection methods, and preparation methods disclosed in the present disclosure are conventional techniques in molecular biology, biochemistry, chromatin structure and analysis, analytical chemistry, cell culture, recombinant DNA technology, and other related fields.

### Embodiment 1

The sequence of an anti-CD19 28Z CAR was referred to the sequence shared by Rosenberg, S.A. on the NCBI. Through the gene synthesis technology, a CD3ε full-length intracellular segment was inserted behind a CD28 TM transmembrane region, and a newly constructed CAR was obtained and named E28Z.

The sequence of the anti-CD19 E28Z CAR is shown as SEQ ID NO: 7, specifically:

ATGGTGAGCAAGGGCGAGGAGCTGTTCACCGGGGTGGTGCCCA
TCCTGGTCGAGCTGGACGGCGACGTAAACGGCCACAAGTTCAGCGTGT
CCGGCGAGGGCGAGGGCGATGCCACCTACGGCAAGCTGACCCTGAAG
TTCATCTGCACCACCGGCAAGCTGCCCGTGCCCTGGCCCACCCTCGTG
ACCACCCTGACCTACGGCGTGCAGTGCTTCAGCCGCTACCCCGACCAC
ATGAAGCAGCACGACTTCTTCAAGTCCGCCATGCCCGAAGGCTACGTC
CAGGAGCGCACCATCTTCTTCAAGGACGACGGCAACTACAAGACCCGC
GCCGAGGTGAAGTTCGAGGGCGACACCCTGGTGAACCGCATCGAGCT
GAAGGGCATCGACTTCAAGGAGGACGGCAACATCCTGGGGCACAAGCT
GGAGTACAACTACAACAGCCACAACGTCTATATCATGGCCGACAAGCAG
AAGAACGGCATCAAGGTGAACTTCAAGATCCGCCACAACATCGAGGAC
GGCAGCGTGCAGCTCGCCGACCACTACCAGCAGAACACCCCCATCGG
CGACGGCCCCGTGCTGCTGCCCGACAACCACTACCTGAGCACCCAGTC
CGCCCTGAGCAAAGACCCCAACGAGAAGCGCGATCACATGGTCCTGCT
GGAGTTCGTGACCGCCGCCGGGATCACTCTCGGCATGGACGAGCTGTA
CAAGGGAAGCGGAGAGGGCAGAGGAAGTCTGCTAACATGCGGTGACG
TCGAGGAGAATCCTGGACCTatgcttctcctggtgacaagccttctgctctgtgagttaccaca
cccagcattcctcctgatcccaTACCCCTACGACGTGCCCGACTACGCCgacatccag
atgacacagactacatcctccctgtctgcctctctgggagacagagtcaccatcagttgcagggcaagtca
ggacattagtaaatatttaaattggtatcagcagaaaccagatggaactgttaaactcctgatctaccatac
atcaagattacactcaggagtcccatcaaggttcagtggcagtgggtctggaacagattattctctcaccatt

agcaacctggagcaagaagatattgccacttacttttgccaacagggtaatacgcttccgtacacgttcgg
aggggggactaagttggaaataacaggctccacctctggatccggcaagcccggatctggcgagggat
ccaccaagggcgaggtgaaactgcaggagtcaggacctggcctggtggcgccctcacagagcctgtcc
gtcacatgcactgtctcaggggtctcattacccgactatggtgtaagctggattcgccagcctccacgaaag
ggtctggagtggctgggagtaatatggggtagtgaaaccacatactataattcagctctcaaatccagact
gaccatcatcaaggacaactccaagagccaagttttcttaaaaatgaacagtctgcaaactgatgacaca
gccatttactactgtgccaaacattattactacggtggtagctatgctatggactactggggtcaaggaacct
cagtcaccgtctcctcagcggccgcaattgaagttatgtatcctcctccttacctagacaatgagaagagca
atggaaccattatccatgtgaaagggaaacacctttgtccaagtcccctatttcccggaccttctaagcccttt
tgggtgctggtggtggttgggggagtcctggcttgctatagcttgctagtaacagtggcctttattattttctgggt
gaagaatagaaaggccaaggccaagcctgtgacacgaggagcgggtgctggcggcaggcaaaggg
gacaaaacaaggagaggccaccacctgttcccaacccagactatgagcccatccggaaaggccagc
gggacctgtattctggcctgaatcagagacgcatcaggagtaagaggagcaggctcctgcacagtgact
acatgaacatgactccccgccgccccgggcccacccgcaagcattaccagccctatgccccaccacgc
gacttcgcagcctatcgctccagagtgaagttcagcaggagcgcagacgcccccgcgtaccagcaggg
ccagaaccagctctataacgagctcaatctaggacgaagagaggagtacgatgttttggacaagagacg
tggccgggaccctgagatggggggaaagccgagaaggaagaaccctcaggaaggcctgtacaatga
actgcagaaagataagatggcggaggcctacagtgagattgggatgaaaggcgagcgccggagggg
caaggggcacgatggcctttaccagggtctcagtacagccaccaaggacacctacgacgcccttcacat
gcaggccctgccccctcgctaa.

The CAR was subcloned to a lentivirus expression plasmid pHAGE vector, and the CAR expression plasmid, the packaging plasmid pSPAX2, and the pMD2G were mixed at a ratio of 10: 7.5: 3.5, afterward, were transferred into 293 FT cells by a calcium phosphate transfection method to produce lentivirus particles. The lentiviruses expressing 28Z CAR or E28Z CAR were concentrated into small-volume high-titer viruses (~10^8/ml) by ultracentrifugation method, and then were used to transfect primary T cells (MOI = 10) activated by αCD3/αCD28 Ab-beads for one day. The primary T cells were cultured with T cell complete medium (XIVO-15+1% P.S.+10ng/ml human IL-7+10ng/ml human IL-15+10mM+5% Human AB serum, 10 mM neutralized NAC), the viruses were removed one day after transfection, and the antibodies were removed four days after stimulation. On the 5th day after antibody stimulation, CAR-T cells with the same CAR expression level were sorted out. After a period of amplification, CAR-T was characterized by a series of indicators, such as surface CAR level, the CD4/CD8 ratio, in vitro cytokine secretion, in vitro killing ability, in vitro proliferation ability, in vitro apoptosis level, in vitro sustained viability, and anti-tumor effect in vivo.

The structures of 28Z CAR and E28Z CAR targeting CD19 are shown in Fig. 1: a.

The CAR level on the membrane surface of the 28Z CAR-T and E28Z CAR-T cells was detected by flow cytometry employing anti-mouse FM 63 scFv coupled with Alexa Fluor 647 antibody. The results of the flow cytometry are shown in Fig. 1:b, indicating there was no difference in CAR positive rate and expression level (MFI) between the 28Z CAR-T and E28Z CAR-T cells.

The 28Z CAR-T and E28Z CAR-T cells were detected by flow cytometry employing anti-human CD4-APC and anti-human CD8-PE-Cy7 antibodies. The results of the flow cytometry are shown in Fig. 1:c, indicating that the ratios of CD4⁺ CAR-T cells to CD8⁺ CAR-T cells of the 28Z CAR-T and E28Z CAR-T cells were similar.

CAR-T cells were mixed with CD19⁺ lymphoma cell line Raji at a ratio of 1:1, then were centrifuged at 4°C to promote the contact of CAR-T cells and CD19⁺ lymphoma cell line, afterward, were placed in a 37°C water bath. The reaction was terminated with LDS sample buffer at a series of time points, and then was detected by anti-pCD3ζ (p142) antibody western blotting. As shown in Fig. 1: d, the pCD3ζ band showed that the basal pCD3ζ phosphorylation level at 0 min (i.e., without the addition of CD19 antigen) of E28Z CAR-T cells was significantly higher than that of 28Z CAR-T cells. After the specific stimulation of CD19 antigen, the pCD3ζ phosphorylation of E28Z CAR-T cells increased first and then decreased, and the decrease rate of pCD3ζ phosphorylation was significantly faster than that of 28Z CAR-T cells.

100,000 CAR-T cells were mixed with CD19+ lymphoma cell line Raji in a 1.5 ml EP tube at a ratio of 1:1, then were centrifuged (400 g, 1 min) at room temperature to promote the contact of CAR-T cells and CD19+ lymphoma cell line, afterward, were cultured with complete medium put in a 37°C incubator. CAR internalization was terminated with cold PBS at a series of time points. The obtained sample was placed in ice and washed once with cold PBS, and then stained (4°C, 30 min) with anti-mouse FM63 scFv coupled with Alexa Fluor647 antibody. The CAR level on the surface membrane of 28Z CAR-T and E28Z CAR-T cells was detected by flow cytometry. As shown in Fig. 1:e, the surface level of 28Z and E28Z CAR-T cells decreased sharply first, and then slowly increased. The surface level of 28Z CAR decreased faster than that of E28Z CAR, and increased significantly faster than that of E28Z CAR subsequently.

100,000 CAR-T cells and CD19⁺ lymphoma cell line Raji were inoculated in a round-bottom 96-well plate at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for one day before sample collection. 1x BFA was added to the sample 6 hours before sample collection to inhibit the efflux of cytokine, and then the collected sample was washed once with PBS, afterward, was fixed at room temperature for 5 min using 4% PFA, then was punched at room temperature for 5 min utilizing 0.1% TritonX-100. IL-2, IFN-γ, and TNF-α were detected by intracellular staining known in the art. As shown in Figs. 1: f-h, the cytokines IL-2, IFN-γ, and TNF-α produced from E28Z CAR-T cells after specific stimulation of CD19⁺ cells were significantly less than those of 28Z CAR-T cells.

Figs. 1d and 1c indicated that the rapid decrease of the pCD3ζ phosphorylation level of E28Z CAR was not caused by the down-regulation of surface CAR level, because the surface level of 28Z CAR decreased faster than that of E28Z CAR after CD19-specific stimulation.

100,000 CAR-T cells and CD19⁺ lymphoma cell line Raji were inoculated in a round-bottom at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for 2 h and 4 h, where anti-human CD107a-eFluor660 antibody and 1×Monensin (to prevent anti-human CD107a antibody from endocytic degradation) were added to the complete medium. The reaction was terminated with cold PBS for sample collection. After the sample was collected, the sample was washed once with PBS. As shown in Fig. 1:i, the degranulation rate of E28Z CAR-T cells after CD19 antigen-specific stimulation was significantly greater than that of 28Z CAR-T cells.

100,000 CAR-T cells and CD19⁺ lymphoma cell line Raji were inoculated in a round-bottom 96-well plate at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for one day. 6 hours before sample collection, 1xBFA was added to inhibit granzyme efflux. After sample collection, the collected sample was washed once with PBS, then fixed for 5 min with 4%PFA at room temperature, and afterward was punched for 5 min with 0.1%TritonX-100 at room temperature. Granzyme B was detected by conventional intracellular staining. As shown in Fig. 1:j, Granzyme B generated from E28Z CAR-T cells after CD19⁺ cell-specific stimulation was significantly more than that of 28Z CAR-T cells.

Cytotoxicity of anti-CD19 28Z and E28Z CAR-T towards CD19⁺ tumor cells. CD19⁻K562 cells were resuspended in 1640 serum-free medium, then pre-stained with 1x CellTraker deep red dye (37°C water bath, 30 min), afterward, were washed with 1640 serum-free medium once, and were resuspended in T-cell complete medium. The resuspended cells were mixed with CD19⁺K562_CD19 (IRES mCherry) cells at a ratio of 1: 1 to serve as target cells. CAR-T cells were mixed with the above target cells at ratios of 3:1, 1:1, and 1:3, respectively, in round-bottom 96-well plates, then mixed well, afterward, were centrifuged (400 g, 1 min) at room temperature to promote the contact of CAR-T cells and target cells. The obtained sample was cultured with complete medium put in a 37°C incubator for one day. The sample was collected and placed in ice. The percentage of CD19⁻K562 cells (APC⁺, mCherry) and the percentage of CD19⁺K562_CD19 cells (APC⁻, mCherry⁺) were detected by flow cytometry, where the percentage of CD19⁻K562 cells (APC⁺, mCherry⁻) / the percentage of CD19⁺K562_CD19 cells (APC⁻, mCherry⁺) in the experimental group was recorded as N. N was standardized with "N₀= the percentage of CD19⁻K562 cells (APC⁺, mCherry) / the percentage of CD19⁺K562_CD19 cells (APC⁻, mCherry⁺)" of the target cells in which no CAR-T cells were added. The survival ratio was calculated as N/N₀ after standardization, and the killing rate formula was "Lysis(%)=1-N/N₀". As shown in Fig. 1:k, the cytotoxicity of the anti-CD19 E28Z CAR-T towards CD19⁺ tumor cells was higher than that of 28Z CAR-T cells.

100,000 CAR-T cells and CD19⁺ lymphoma cell line Raji were inoculated in a round-bottom 96-well plate at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for a period of time. After sample collection at different time points, the samples were washed once with PBS, respectively. According to the instructions of the Foxp3/Transcription Factor Staining Buffer Set Kit, the expression level of Ki67 was detected by flow cytometry after anti-human Ki67-APC antibody staining. As shown in Fig. 1:1, anti-CD19 E28Z CAR-T proliferated faster than that of 28Z CAR-T after being stimulated by CD19⁺ Raji cells for a period of time.

100,000 CAR-T cells and CD19⁺ lymphoma cell line Raji were inoculated in a round-bottom 96-well plate at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for a period of time. After sample collection at different time points, the samples were washed once with PBS, respectively. According to the instructions of the Annexin V Apoptosis Detection Kit APC Kit, the Annexin V expression level of CAR-T cells was detected by flow cytometry after staining. As shown in Fig. 1:m, the apoptosis level of anti-CD19 E28Z CAR-T stimulated by CD19⁺ Raji cells for a period of time was significantly lower than that of 28Z CAR-T.

100,000 CAR-T cells and CD19⁺ lymphoma cell line Raji were inoculated in a round-bottom 96-well plate at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for a period of time. The samples were washed once with PBS, respectively, after sample collection at different time points, and the survival amount of CAR⁺T cells was detected by anti-human CD4-APC and anti-human CD8-PE-Cy7 antibody staining. As shown in Fig. 1:n, the survival amount of anti-CD19 E28Z CAR-T after stimulation with CD19⁺ Raji cells was significantly higher than that of 28Z CAR-T.

100,000 CAR-T cells and CD19⁺ lymphoma cell line Raji were inoculated in a round-bottom 96-well plate at a ratio of 1:1, then mixed well, afterward, were centrifuged (400 g, 1 min) at room temperature to promote the contact of CAR-T cells and CD19⁺ lymphoma cell line. The obtained sample was cultured with complete medium put in a 37°C incubator for a period of time. The samples were washed once with PBS, respectively, after sample collection at different time points. The PD-1 and TIM3 expression of CAR⁺T cells were detected by anti-human PD-1-APC and anti-human TIM3-PE antibody staining. As shown in Figs. 1: o-p, the PD-1 and TIM3 expression of the anti-CD19 E28Z CAR-T after being stimulated by CD19⁺ Raji cells were lower than those of 28Z CAR-T after the 7th day, indicating that E28Z CAR did not cause the exhaustion state of T cells, even the basal pCD3ζ phosphorylation level of the E28Z CAR is high (Fig. 1d).

3×10^7/ml Raji cell-PBS resuspension was prepared by sterile operation. 100 ul Raji cells (3 million) were inoculated subcutaneously to the left back of each 6-week-old B-NDV female mouse, and the time of inoculation was denoted as day0. After 6 days, the diameter of the Raji subcutaneous tumor became 7-8 mm. The mice then were divided into groups randomly, afterward, 100 ul CAR-T cells (8 million) were injected into each mouse through the tail vein. The model is shown in Fig. 1: q. The tumor size was measured regularly with a vernier caliper, and the corresponding data was recorded, where the tumor area = the length × the width. As shown in Fig. 1: r, the tumor of mice in the vector control group grew fastest, followed by the 28Z group, the E28Z group had the best therapeutic effect, and the therapeutic effect of the E28Z group was significantly higher than that of the 28Z group.

### Embodiment 2

As shown in Fig. 2:a, the FMC63 scFv (anti-CD19) sequences of 28Z CAR and E28Z CAR targeting CD19 were sequentially replaced with SS1 scFv (anti-mesothelin), therefore, the 28Z CAR and E28Z CAR targeting mesothelin can be obtained.

The CAR level on the membrane surfaces of the 28Z and E28Z CAR-T cells was detected by flow cytometry with primary antibody Rat anti-HA and secondary antibody goat anti-Rat IgG-Alexa Fluor647 antibody. The results of flow cytometry are shown in Fig. 2: b, indicating that there was no difference in the CAR positive rate and expression level (MFI) between the 28Z CAR-T and E28Z CAR-T cells.

Anti-mesothelin 28Z CAR-T and E28Z CAR-T cells were detected by flow cytometry with anti-human CD4-APC and anti-human CD8-PE-Cy7 antibodies. The results of flow cytometry are shown in Fig. 2:c. c, indicating that the ratios of CD4⁺ CAR-T cells to CD8⁺ CAR-T cells of the 28Z CAR-T and E28Z CAR-T cells were similar.

100,000 anti-mesothelin CAR-T cells and the K562-Mesothelin cell line overexpressing mesothelin were inoculated into a round-bottom 96-well plate at a ratio of 1: 1, then mixed well, afterward, were centrifuged (400 g, 1 min) at room temperature to promote the contact of CAR-T cells and K562-Mesothelin cell line. The obtained sample was cultured with complete medium put in a 37°C incubator for one day. 6 hours before sample collection, 1x BFA was added to inhibit cytokine efflux. Afterward, the sample was collected and then washed once with PBS, then fixed for 5 min with 4%PFA at room temperature, and punched for 5 min with 0.1%TritonX-100 at room temperature. IL-2, IFN-γ, and TNF-α were detected by conventional intracellular staining. As shown in Figs. 2: d-f, the cytokines IL-2, IFN-γ, and TNF-α generated by the anti-mesothelin E28Z CAR-T cell after the specific stimulation of K562-Mesothelin cells were significantly less than those of the anti-mesothelin 28Z CAR-T cell.

100,000 CAR-T cells and the K562-Mesothelin cell line overexpressing mesothelin were inoculated into a round-bottom 96-well plate at a ratio of 1: 1, then mixed well, afterward, were centrifuged (400 g, 1 min) at room temperature to promote the contact of CAR-T cells and K562-Mesothelin cell line. The obtained sample was cultured with complete medium put in a 37°C incubator for a period of time. After sample collection at different time points, the samples were washed once with PBS, respectively. The Annexin V expression level of CAR-T cells was detected by flow cytometry after staining according to the instructions of Annexin V Apoptosis Detection Kit APC Kit. As shown in Fig. 2: g, the apoptosis level of the anti-mesothelin E28Z CAR-T after stimulation with the K562-mesothelin cell line for a period of time was significantly lower than that of the anti-mesothelin 28Z CAR-T.

100,000 CAR-T cells and K562-Mesothelin cell line overexpressing mesothelin were inoculated into a round-bottom 96-well plate at a ratio of 1: 1, then mixed well, afterward, were centrifuged (400 g, 1 min) at room temperature to promote the contact of CAR-T cells and K562-Mesothelin cell line. The obtained sample was cultured with complete medium put in a 37°C incubator for a period of time. After sample collection at different time points, the samples were washed once with PBS, respectively. The survival number of CAR⁺T cells was detected by anti-human CD4-APC and anti-human CD8-PE-Cy7 antibody staining. As shown in Fig. 2: h, the survival number of the anti-mesothelin E28Z CAR-T after stimulation with the K562-mesothelin cell line was significantly higher than that of the 28Z CAR-T.

The mesothelin-K562 cells were resuspended in a 1640 serum-free culture medium, then pre-stained with 1x CellTraker deep red dye (37°C water bath, 30 min), afterward, were washed once with 1640 serum-free culture medium, and were resuspended in T-cell complete medium. The resuspended cells were mixed with mesothelin⁺K562_mesothelin (IRES mCherry) cells at a ratio of 1: 1 to serve as target cells. CAR-T cells were mixed with the above target cells at ratios of 3:1, 1:1, and 1:3, respectively, in round-bottom 96-well plates, then mixed well, afterward, were centrifuged (400 g, 1 min) at room temperature to promote the contact of CAR-T cells and target cells. The obtained sample was cultured with complete medium put in a 37°C incubator for one day. The samples were collected and placed in ice. The percentage of mesothelin⁻K562 cells (APC⁺, mCherry) and the percentage of mesothelin⁺K562_mesothelin cells (APC⁻, mCherry⁺) were detected by flow cytometry, where the percentage of mesothelin⁻K562 cells (APC⁺, mCherry) / the percentage of mesothelin⁺K562_mesothelin cells (APC⁻, mCherry⁺) in the experimental group was recorded as N. N was normalized by "N₀= the percentage of the mesothelin-K562 cell (APC⁺, mCherry) / the percentage of the mesothelin⁺K562_mesothelin cell (APC⁻, mCherry⁺) " of the target cells in which no CAR-T cells were added. The survival ratio was calculated as N/N₀ after normalization, and the killing rate formula was "Lysis (%) =1-N/N₀". As shown in Fig. 2: i, the cytotoxicity of the anti-mesothelin E28Z CAR-T towards mesothelin+ tumor cells was greater than that of the 28Z CAR-T cell.

2×10^7/ml PANC-1 cell-PBS resuspension was prepared by sterile operation. 100ul PANC-1 cells (2 million) were inoculated subcutaneously to the left back of each 6-week-old B-NDG female mouse, and the time of inoculation was recorded as day0. After 7 days, the diameter of the PANC-1 subcutaneous tumor became 7-8 mm. The mice then were divided into groups randomly, afterward, 100 ul of anti-mesonthein CAR-T cells (6 million) were injected into each mouse tough the tail vein. The model is shown in Fig. 2: j. The tumor size was measured regularly with a vernier caliper, and the corresponding data was recorded, where the tumor area = the length × the width. As shown in Fig. 2: k, the tumors of mice in the vector control group and the experimental group (i.e., 28Z group) grew fastest, and there was no difference between the above two groups. The E28Z group had a relatively better treatment effect, and there was a statistical difference.

Experimental results:
(1) The surface levels and the CD4/CD8 ratios of the E28Z and 28Z CAR-T cells were similar.
(2) After the CAR-T cells were specifically stimulated by Raji cells in vitro, the cytokines (IFN-γ, IL-2, and TNF) secreted by the E28Z was significantly lower than those of the 28Z, while GrzB generated from the E28Z was significantly higher than that of the 28Z.
(3) After the CAR-T cells were specifically stimulated by Raji cells in vitro, the surface CAR (%) of the 28Z decreased faster than that of the E28Z in a short initial time, and then the surface CAR (%) increased significantly faster than that of the E28Z.
(4) After the CAR-T cells were specifically stimulated by Raji cells in vitro and amplified for a period of time, the level of apoptosis was detected at different time points. The apoptosis levels of the 28Z and the E28Z were similar at first, and then the apoptosis level of the E28Z was significantly lower than that of the 28Z.
(5) After the CAR-T cells were specifically stimulated by Raji cells in vitro and amplified for a period of time, the CAR-T cells were sampled and detected at different time points, and the proliferation capacity (namely, Ki67 expression level) of the E28Z was significantly higher than that of the 28Z.
(7) After the CAR-T cells were specifically stimulated by Raji cells and amplified for a period of time, the number of the amplified CAR + T cells was detected at different time points, and the number of the E28Z was significantly more than that of the 28Z.
(8) In the in vitro killing experiment, where CAR-T cells were co-incubated with CD19+K562:CD19-K562, the lethality of the E28Z with the higher dose was significantly greater than that of the 28Z.
(9) In the in vivo anti-tumor experiments of mice, the curative effect of the E28Z was significantly better than that of the 28Z.

The above are merely preferred embodiments of the present disclosure, and are not limitations to the form and essence of the present disclosure. It should be noted that for a person of ordinary skill in the art, a number of improvements and supplements can be made without departing from the method of the present disclosure, and these improvements and supplements should also be regarded as the protection scope of the present disclosure. A person skilled in the art, without deviating from the spirit and scope of the present disclosure, can make some equivalent changes of alteration, modification, and variation according to the technical content disclosed above, which should be regarded as the equivalent embodiments of the present disclosure. And any equivalent changes, modifications, and variations to the above embodiments according to the essential technology of the present disclosure still fall within the scope of the technical solutions of the present disclosure.

## Claims

1. A chimeric antigen receptor, comprises:
an extracellular domain, a transmembrane domain, and an intracellular domain which are connected in sequence,
wherein the extracellular domain comprises an antigen recognition region and a hinge region;
and one end of the intracellular domain which is connected to the transmembrane domain is connected to a CD3ε intracellular region.

2. The chimeric antigen receptor according to claim 1, further comprises one or more of the following:
(1) the amino acid sequence of the CD3ε intracellular region is shown as SEQ ID NO: 1;
(2) the intracellular domain comprises the CD3ε intracellular region, a costimulatory signaling region, and a CD3ζ intracellular region that are sequentially connected.

3. The chimeric antigen receptor according to claim 2, wherein the costimulatory signaling region is selected from one or more of intracellular regions of CD27, CD28, CD134, 4-1BB, and ICOS.

4. The chimeric antigen receptor according to claim 3, further comprises one or more of the following:
(1) the amino acid sequence of the CD28 intracellular region is shown as SEQ ID NO: 2;
(2) the amino acid sequence of the CD3ζ intracellular region is shown as SEQ ID NO: 3.

5. The chimeric antigen receptor according to claim 1, further comprises one or more of the following:
a. the antigen recognition region is selected from a single-chain antibody against a tumor surface antigen, and the tumor surface antigen is selected from one or more of CD19, mesothelin, CD20, CD22, CD123, CD30, CD33, CD38, CD138, BCMA, Fibroblast activation protein, Glypican-3, CEA, EGFRvIII, PSMA, Her2, IL13Rα2, CD 171 and GD2;
b. the transmembrane domain is selected from CD28TM, CD4, CD8α, OX40, and H2 -Kb.

6. The chimeric antigen receptor according to claim 5, further comprises one or more of the following:
c. the single-chain antibody is selected from FMC63 and SS1;
d. the amino acid sequence of CD28 TM is shown as SEQ ID NO: 4.

7. The chimeric antigen receptor according to any one of claims 1-6, wherein the amino acid sequence of the chimeric antigen receptor is shown as SEQ ID NO: 5 or SEQ ID NO: 6.

8. A polynucleotide sequence, wherein the polynucleotide sequence is selected from:
(1) a polynucleotide sequence encoding the chimeric antigen receptor according to any one of claims 1-7; and
(2) a complementary sequence to the polynucleotide sequence in (1).

9. The polynucleotide sequence according to claim 8, wherein the polynucleotide sequence is shown as SEQ ID NO: 7 or SEQ ID NO: 8.

10. A nucleic acid construct, comprises the polynucleotide sequence according to any one of claims 8-9;
preferably, the nucleic acid construct is a vector;
more preferably, the nucleic acid construct is a lentivirus vector, which contains a replication initiation site, a 3' LTR, a 5' LTR, and the polynucleotide sequence according to any one of claims 8-9.

11. A lentivirus vector system, comprises the nucleic acid construct according to claim 10 and a lentivirus vector auxiliary component.

12. A genetically modified T cell, comprises the polynucleotide sequence according to any one of claims 8-9 or the nucleic acid construct according to claim 10, or infects the lentivirus vector system according to claim 11.

13. A use of the chimeric antigen receptor according to any one of claims 1-7, the polynucleotide sequence according to any one of claims 8-9, the nucleic acid construct according to claim 10, or the lentivirus vector system according to claim 11 in any one or more of the following:
(1) preparing T cells; (2) inhibiting T cells from secreting cytokines IFN-γ, IL-2, and TNF; (3) inhibiting T cell apoptosis; (5) enhancing T cell proliferation capacity; (6) improving T cell lethality; (7) promoting GrzB generation; and (8) promoting degranulation.

14. A use of the chimeric antigen receptor according to any one of claims 1-7, the polynucleotide sequence according to any one of claims 8-9, the nucleic acid construct according to claim 10, the lentivirus vector system according to claim 11, or the genetically modified T cell according to claim 12 in any one or more of the following:
(1) treating tumors; (3) inhibiting a generation of cytokine storm during tumor treatment; (4) inhibiting a generation of neurotoxicity during tumor treatment;
preferably, the tumor is selected from leucocythemia or one or more of solid tumors.
